(19) Europäisches Patentamt
European Patent Office
Office européen des brevets

(11) Veröffentlichungsnummer : **0 167 872 B1**

(12) # EUROPÄISCHE PATENTSCHRIFT

(45) Veröffentlichungstag der Patentschrift : **28.03.90**

(51) Int. Cl.[5] : **C 07 C209/16**, C 07 C211/02, C 07 C211/09, C 07 C213/02

(21) Anmeldenummer : **85107313.0**

(22) Anmeldetag : **13.06.85**

(54) **Verfahren zur Herstellung von Aminen.**

(30) Priorität : **19.06.84 DE 3422610**

(43) Veröffentlichungstag der Anmeldung : **15.01.86 Patentblatt 86/03**

(45) Bekanntmachung des Hinweises auf die Patenterteilung : **28.03.90 Patentblatt 90/13**

(84) Benannte Vertragsstaaten : **DE FR GB IT NL SE**

(56) Entgegenhaltungen :
**EP–A– 0 006 454**
**EP–A– 0 009 590**
**EP–A– 0 024 225**
**EP–A– 0 127 874**
**US–A– 4 014 933**

(73) Patentinhaber : **BASF Aktiengesellschaft**
**Carl-Bosch-Strasse 38**
**D-6700 Ludwigshafen (DE)**

(72) Erfinder : **Franzischka, Wolfgang, Dr. Dipl.-Ing.**
**Franz-Lind-Strasse 1**
**D-6713 Freinsheim (DE)**
Erfinder : **Koernig, Wolfgang, Dr.-Chem.**
**Dachsweg 5**
**D-6901 Dossenheim (DE)**
Erfinder : **Schroeder, Wolfgang, Dr.-Chem.**
**Seebacher Strasse 51**
**D-6702 Bad Duerkheim (DE)**

**EP 0 167 872 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Verfahren zur Herstellung von Aminen mit einer oder zwei endständigen Dimethylaminogruppen durch Umsetzung von gegebenenfalls substituierten Alkandiolen oder N,N-Dimethylaminoalkanolen mit Dimethylamin in der Gasphase bei einem Druck von 5 bis 30 bar.

Es ist bekannt, Diole mit Dialkylaminen zu den entsprechenden Dialkylaminoverbindungen umzusetzen. So beschreibt die DE-A-2 824 908 ein Verfahren zur Herstellung von Bis(dialkylamino)alkanen, -oxaalkanen und -azaalkanen. Dabei werden die jeweiligen Diole mit einem Dialkylamin in Gegenwart von Kupfer und Chrom enthaltenden Katalysatoren bei einer Temperatur von 150 bis 250 °C in flüssiger Phase umgesetzt. Dialkylamin muß dabei in 2- bis 10-fachem stöchiometrischem Überschuß verwendet werden. Wie sich aus den Beispielen ergibt, muß außerdem zur Durchführung der Umsetzung ein hoher Druck aufgewendet werden (40 bzw. 250 bar). Zur Erzielung befriedigender Ausbeuten sind schließlich zudem hohe Verweilzeiten im Reaktor erforderlich (2 bis 4 Stunden).

Nach der Lehre der DE-A-3 104 738 lassen sich Alkandiole, Etherdiole, N,N-Dimethylaminoalkanole oder Azaalkanole mit Dimethylamin in der Gasphase an einem Kupfer-Katalysator zu den entsprechenden permethylierten Aminen oder gegebenenfalls zu deren Zwischenstufen, den jeweiligen N,N-Dimethylaminoalkanolen, umsetzen.

Um bei diesem Verfahren zufriedenstellende Ausbeuten an den jeweils gewünschten Wertprodukten zu erhalten, ist es notwendig bei atmosphärischem Druck zu arbeiten. Schon geringe Druckerhöhungen, beispielsweise bis zu 3 bar, führen zu vermehrter Bildung von Nebenprodukten.

In einigen Fällen, insbesondere bei der Umsetzung von Dimethylamin mit Diethylenglykol, N,N-Dimethylaminoethanol oder 1,4-Butandiol, entstehen undefinierte hochsiedende Substanzen, deren Menge bis zu 30 %, bezogen auf die jeweils zu erwartenden Endprodukte, betragen kann.

Neben der unerwünschten Ausbeuteverminderung beeinträchtigen diese hochsiedenden Substanzen außerdem die Reinheit der Zielprodukte. Aufgrund ihrer thermischen Instabilität zerfallen sie nämlich während der Destillation des Reaktoraustrags zu Spaltproduken und geben somit Anlaß zu zusätzlichen aufwendigen Reinigungsoperationen bei der Aufarbeitung.

Aufgabe der vorliegenden Erfindung war es nun, ein Verfahren bereitzustellen, das die geschilderten Nachteile des Standes der Technik vermeidet.

Es wurde gefunden, daß man Amine der allgemeinen Formel I

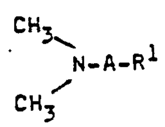

(I)

in der $R^1$ eine Hydroxylgruppe oder Dimethylaminogruppe und A eine Alkylengruppe mit 2 bis 6 Kohlenstoffatomen bedeuten, wobei die Kohlenstoffatomkette der Alkylengruppe gegebenenfalls durch Sauerstoffatome oder $N—R^2$-Gruppen, in denen $R^2$ für ein Wasserstoffatom oder eine niedere Alkylgruppe steht, unterbrochen sein kann, durch Umsetzung von Alkoholen der allgemeinen Formel II

$$HO—A—R^1 \quad (II)$$

in der $R^1$ und A die oben genannte Bedeutung besitzen, mit Dimethylamin in der Gasphase an einem kupferhaltigen Katalysator vorteilhaft erhält, wenn man die Umsetzung bei einer Temperatur von 160 bis 240 °C und bei einem Druck von 5 bis 30 bar durchführt und dabei ein Molverhältnis Dimethylamin : Moläquivalent OH-Gruppen von 0,5 : 1 bis 3 : 1 einhält.

Geeignete Alkohole, die der Formel II gehorchen, sind einerseits Alkandiole mit 2 bis 6 Kohlenstoffatomen, z. B. 1,2-Ethandiol, 1,4-Butandiol, 1,5-Pendandiol oder 1,6-Hexandiol.

Andererseits sind auch Oxaalkandiole bzw. Azaalkandiole geeignet. Bei diesen Verbindungsklassen ist die Kohlenstoffatomkette der genannten Alkandiole durch Sauerstoffatome bzw. Iminogruppen unterbrochen, wobei das Wasserstoffatom der Iminogruppen auch durch niedere Alkylreste, wie den Methyl-, Ethyl- oder Propylrest ersetzt sein kann. Beispielsweise sind die Oxaalkandiole Diethylenglykol, Triethylenglykol oder Dipropylenglykol bzw. die Azaalkandiole Diethanolamin, N-Methyldiethanolamin, N-Ethyldiethanolamin, Dipropanolamin oder N-Methyldipropanolamin zu nennen.

Schließlich sind auch alle diejenigen N,N-Dimethylaminoalkanole für das erfindungsgemäße Verfahren geeignet, die sich von den oben genannten Diolen dadurch ableiten, daß eine alkoholische Hydroxylgruppe durch eine Dimethylaminogruppe ersetzt ist.

Die Produkte der zuletzt genannten Klasse können im erfindungsgemäßen Verfahren sowohl als Ausgangsprodukte wie auch als Zwischen- oder Endprodukte betrachtet werden.

Als Ausgangsprodukte sind sie anzusehen, wenn sie durch die Umsetzung mit Dimethylamin in die entsprechenden Bis(N,N-dimethylamino)-verbindungen übergeführt werden sollen.

Als Zwischenprodukte sind sie zu betrachten, wenn sie bei der Umsetzung der entsprechenden Diole

mit Dimethylamin zu den Bis(N,N-dimethylamino)-verbindungen intermediär als Mono(N,N-dimethylamino)-verbindungen entstehen und durch Weiterreaktion mit Dimethylamin in die jeweiligen Bisverbindungen übergeführt werden.

Als Endprodukte sind sie schließlich zu behandeln, wenn die Umsetzung der entsprechenden Diole mit Dimethylamin gezielt auf die Klasse der N,N-Dimethylaminoalkanole gerichtet ist.

Die zu verwendenden Katalysatoren sind üblicherweise Trägerkatalysatoren und enthalten als aktive Komponente praktisch nur Kupfer. Im allgemeinen beträgt der Kupfergehalt im Katalysator mehr als 20 Gew.%, bezogen auf den Katalysator. Das Kupfer kann auf einen inerten Träger z. B. durch Tränken mit einer Kupfersalzlösung aufgebracht werden oder auch gemeinsam mit dem Inertmaterial aus einer vereinigten Salzlösung gefällt werden. Als Inertmaterial eignet sich z. B. poröse Kieselsäure, Erdalkalisilikat, Aluminiumoxid. Weiterhin können auch basische Zusätze, beispielsweise Erdalkalioxide im Katalysator enthalten sein.

Besonders bevorzugt ist dabei ein Katalysator, der durch Temperung eines basischen Kupfer und Aluminium enthaltenden Carbonats der allgemeinen Zusammensetzung

$$Cu_mAl_6(CO_3)_{0,5m}O_3(OH)_{M+12} ,$$

wobei m einen beliebigen, auch nicht ganzzahligen Wert zwischen 2 und 6 bedeutet, bei einer Temperatur von 350 bis 700 °C erhältlich ist. Die Herstellung von solchen basischen Kupfer und Aluminium enthaltenden Carbonaten ist in der DE-B-2 445 303 beschrieben.

Die getemperte Verbindung kann durch Mahlen und Aussieben auf eine geeignete Korngrößenverteilung in eine katalytisch besonders aktive Form übergeführt werden. Sie läßt sich aber auch durch bekannte Methoden in die Form von Strängen, Pillen, Kugeln, Ringen etc. bringen. Ihre Aktivierung erfolgt durch Reduktion, wobei man die Reduktion zweckmäßig in einer der eigentlichen Umsetzung vorgelagerten Stufe vornimmt. Als Reduktionsgas verwendet man zweckmäßig ein Wasserstoff/Stickstoff-Gemisch, das 5 bis 10 Vol.% Wasserstoff enthält. Die Reduktionstemperatur beträgt 140 bis 200 °C. Die Reduktion erfolgt bevorzugt bei etwa atmosphärischem Druck.

Für eine optimale Durchführung des erfindungsgemäßen Verfahrens ist es erforderlich, daß ein Druck von 5 bis 30 bar, vorzugsweise 8 bis 25 bar, eingehalten wird.

Außerdem müssen die Reaktionspartner in gasförmigem Zustand und in vollständiger Vermischung zur Reaktion gebracht werden, wobei das Molverhältnis Dimethylamin : Moläquivalent OH-Gruppen 0,5 : 1 bis 3 : 1, vorzugsweise 1 : 1 bis 1,5 : 1 zu betragen hat. Der Wert von 3 : 1 sollte nicht überschritten werden, weil dann durch die Entstehung erheblicher Mengen von Nebenprodukten die Ausbeute deutlich vermindert wird, ohne daß der Umsatz der Reaktion dabei gesteigert würde.

Es ist weiterhin erforderlich, bei einer Temperatur von 160 bis 240 °C, vorzugsweise 180 bis 220 °C und insbesondere von 200 bis 220 °C zu arbeiten.

Durch Einhaltung der geschilderten Maßnahmen gelingt es, die Entstehung der eingangs genannten störenden hochsiedenden Nebenprodukte praktisch völlig zu vermeiden. Zudem kann auch die Entstehung anderer, an sich bekannter Nebenprodukte (z. B. N-methylierte cyclische Imine) mittels des erfindungsgemäßen Verfahrens auf sehr geringem Niveau gehalten werden. Diese Ergebnisse waren überraschend und nicht vorhersehbar. Besonders überraschend ist es, daß mit steigendem Synthesedruck die Raumzeit-Ausbeute entscheidend verbessert wird.

Das erfindungsgemäße Verfahren wird vorteilhaft in einem Rohrenreaktor durchgeführt, wobei sich der Katalysator im allgemeinen innerhalb der Rohre befindet, die üblicherweise einen Durchmesser von 4 bis 10 cm, vorzugsweise 4 bis 6 cm aufweisen.

Es empfiehlt sich, das erfindungsgemäße Verfahren in Anwesenheit eines Trägergases durchzuführen. Als Trägergas verwendet man vorzugsweise Wasserstoff. Die Gasmischung Dimethylamin/Wasserstoff wird im folgenden als « Kreisgas » bezeichnet ; sie enthält ca. 1 bis 10 Vol.%, insbesondere 1 bis 5 Vol.%, Dimethylamin.

Das erfindungsgemäße Verfahren wird zweckmäßig so durchgeführt, daß man die Alkoholkomponente zunächst in einem Vorerhitzer erwärmt und anschließend mit dem Kreisgas, das in einem separaten Vorerhitzer ebenfalls erwärmt wird, vermischt. Diese Mischung tritt dann in einen Verdampfer ein, wo die Alkoholkomponente vollständig verdampft wird. Die resultierende Gasmischung wird dabei auf eine Temperatur gebracht, die mit der im Reaktor benötigten Reaktionstemperatur vergleichbar ist. Zweckmäßig wählt man aber eine etwas höhere Temperatur im Verdampfer, um so eventuelle Wärmeverluste auf dem Weg zum Reaktor auszugleichen.

Die Mengen an Kreisgas und Alkoholkomponente sind so aufeinander abgestimmt, daß der Partialdruck der Alkoholkomponente bei der im Verdampfer herrschenden Temperatur ca. 30 bis 80 % des Sättigungswertes erreicht. Damit ist gewährleistet, daß die Alkoholkomponente vollständig verdampft und nicht etwa in flüssigem Aggregatzustand in den Reaktor eintritt.

Das Verhältnis von Gasmenge zu Reaktorvolumen ist im allgemeinen so bemessen, daß die mittlere Verweilzeit des Gasgemisches im Reaktor kleiner als 30 Sekunden ist. Dabei wird unter dem je Zeiteinheit durchströmendem Gasvolumen $V_G$ dasjenige Volumen verstanden, das sich aus Normalvolumen (z. B. gemessen in $Nm^3$ dividiert durch den Absolutdruck p errechnet. Unter dem Reaktorvolumen $V_R$ wird der leer gedachte Reaktorraum verstanden. Die mittlere Verweilzeit t ist also

$$t = \frac{p \cdot V_R}{V_G} < 30 \text{ Sekunden}$$

Bei Berücksichtigung der Temperaturausdehnung des Gases und der Raumerfüllung des Reaktorraums durch den Katalysator ist die mittlere Verweilzeit aber wesentlich kleiner. Sie ist meistens kleiner als 10 Sekunden.

Diese kurze Verweilzeit reicht aus, um bei einem Durchgang durch den Reaktor einen guten Umsatz zu erzielen und gleichzeitig die Bildung von Nebenprodukten weitgehend zu verhindern.

Das den Reaktor verlassende Gasgemisch wird kondensiert, wobei sich die jeweiligen Reaktionsprodukte abtrennen lassen. Das Kreisgas bleibt dabei im gasförmigem Aggregatzustand. Sein Gehalt an Dimethylamin beträgt an dieser Stelle 1 bis 5 Vol.%. Da diese Konzentration an Dimethylamin relativ gering ist, löst sich auch entsprechend wenig Dimethylamin (ca. 5 Gew.%) im Reaktionskondensat.

Vom Kreisgas wird ein kleiner Anteil, im allgemeinen weniger als 5 Vol.% der stündlich im Kreis geförderten Menge, als Abgas abgezogen, um so den Anteil gasförmiger Nebenprodukte gering zu halten. Die Hauptmenge wird mittels einer Kreisgaspumpe zum Vorerhitzer zurückgefördert. Auf dem Wege dorthin werden die Verluste an Wasserstoff und Dimethylamin ergänzt, um so den Gaskreislauf zu schließen.

Die durch Kondensation abgetrennten Reaktionsprodukte können durch Destillation bei vermindertem Druck in hoher Reinheit erhalten werden.

Dabei ist anzumerken, daß die Destillate unvollständig methylierte Diamine enthalten können, hauptsächlich trimethylierte Diamine. Ihr Gesamtgehalt im Reindestillat liegt im allgemeinen unter 1 Gew.%. Dieser Anteil ist aber um so höher, je höher der Dimethylaminüberschuß in der Synthese ist.

Die genannten trimethylierten Produkte können durch Destillation allerdings nicht abgetrennt werden. Für den Fall, daß besonders reine Zielprodukte gewünscht werden, müssen also diese Nebenprodukte noch entfernt werden. Dabei hat es sich bewährt, dem dimethylaminfreien Rohprodukt ein Carbonsäureanhydrid hinzuzufügen, z. B. Essigsäureanhydrid oder Phthalsäureanhydrid, und zwar in einer Menge von 1 Mol Säureanhydrid pro Mol Trimethyldiaminoalkan. Die Verunreinigung wird so in einen hochsiedenden Stoff umgewandelt, der bei der Destillation des Zielproduktes nicht mehr stört.

Wie oben schon erwähnt ist die Bildung der jeweiligen Bis(N,N-dimethylamino)-verbindungen besonders günstig, wenn als Reaktionspartner das entsprechende, N,N-Dimethylaminoalkanol verwendet wird. In einigen Fällen ist dieses Produkt auch durch eine unabhängige Reaktion vorteilhaft erhältlich. (N,N-Dimethylaminoethanol ist z. B. durch Umsetzung von Ethylenoxid mit Dimethylamin leicht zu gewinnen.) In anderen Fällen ist diese Verbindungsklasse nicht ohne weiteres zugänglich. Sie entsteht jedoch bei der Reaktion des entsprechenden Diols mit Dimethylamin in jedem Falle als Zwischenprodukt.

Durch Einstellen passender Reaktionsbedingungen kann man erreichen, daß das N,N-Dimethylaminoalkanol als Reaktionsprodukt überwiegt. Man erreicht dies z. B. durch Absenken der Reaktionstemperatur, durch Verkürzen der Verweilzeit, geringeres Angebot an Dimethylamin oder durch Kombinieren dieser Maßnahmen.

Die Bis(N,N-dimethylamino)-verbindungen können z. B. als Katalysatoren bei der Polyurethanherstellung Verwendung finden.

Die Mono(N,N-dimethylamino)-verbindungen sind beispielsweise wertvolle Zwischenprodukte bei der Farbstoffherstellung. Sie können aber auch mit einer geeigneten Menge des entsprechenden Diols vermischt werden und erneut in den Reaktionskreislauf zurückgeführt werden.

Die nachfolgenden Beispiele sollen die Erfindung näher erläutern.

### Beispiel 1

. 1,2-Bis(dimethylamino)ethan

Für diese Synthese diente N,N-Dimethylaminoethanol als aus Ausgangsprodukt.

Die Reaktion wurde in einem Röhrenreaktor mit einem inneren Durchmesser von 4 cm und einer Gesamtlänge von 200 cm durchgeführt. Im Inneren des Rohres befand sich, zwischen zwei Schichten aus metallischen Raschigringen, die Katalysatorfüllung. Sie bestand aus 2 l eines Kupferkatalysators (nichtreduzierte Form) und war gemäß DE-B-2 445 303 hergestellt worden. Der Reaktor war mit einem Mantelrohr umgeben, durch das entsprechend erwärmtes Wärmeträgeröl geführt wurde.

Der Katalysator wurde zunächst bei einer Temperatur von 140 bis 200 °C mit einem durch den Reaktor strömenden Wasserstoff/Stickstoff-Gemisch, das 5 bis 10 Vol.% Wasserstoff enthielt, reduziert.

Nach beendeter Reduktion wurden folgende Reaktionsbedingungen eingestellt:

| | |
|---|---|
| Gesamtdruck: | 9 bar |
| Reaktionstemperatur: | 190 °C |
| Kreisgasmenge: | 130 Druckliter pro l Katalysator und Stunde |
| Kreisgaszusammensetzung vor dem Reaktor: | 6 Vol.% Dimethylamin<br>94 Vol.% Wasserstoff |

Dann wurden 0,12 l/l Katalysator und Stunde N,N-Dimethylaminoethanol durch den auf 190 °C erwärmten Vorerhitzer gepumpt und mit dem Kreisgas gemischt. Diese Mischung wurde dem Verdampfer zugeführt, in dem das N,N-Dimethylaminoethanol vollständig verdampft wurde. Das den Verdampfer verlassende Gasgemisch besaß eine Temperatur von 200 °C und wurde nun durch den Reaktor geleitet, wo die Umsetzung unter praktisch isothermen Reaktionsbedingungen stattfand.

Der Umsatz des N,N-Dimethylaminoethanols pro Durchgang war 55 % ; die Ausbeute an 1,2-Bis(N,N-Dimethylamino)ethanol betrug 94 %.

Beispiel 2 (Vergleich)

Man führte die Umsetzung analog Beispiel 1, jedoch bei atmosphärischem Druck durch. Der Umsatz lag bei 50 % ; die Ausbeute an 1,2-Bis(N,N-dimethylamino)ethanol betrug jedoch nur 60 %.

Beispiel 3

Bis(dimethylamino)diethylether und N,N-Dimethylaminodiglykol
Es wurde die Anordnung des Beispiels 1 verwendet und wie folgt betrieben :

| | |
|---|---|
| Gesamtdruck : | 7 bar |
| Reaktionstemperatur | 205 °C |
| Kreisgasmenge | 360 Druckliter/l Katalysator und Stunde |
| Dimethylamingehalt im Kreisgas | |
| vor dem Reaktor : | 5 bis 7 Vol.% |
| nach dem Reaktor : | 2 bis 3 Vol.% |
| Zulauf an Diglykol : | 0,3 l/l Katalysator und Stunde |

Das Kondensat enthielt (ohne Dimethylamin und Reaktionswasser) 35 Gew.% Bis(N,N-dimethylamino)diethylether, 55 Gew.% N,N-Dimethylaminodiglykol, 1 Gew.% nicht umgesetztes Diglykol sowie insgesamt 9 Gew.% mehrere nicht identifizierte Nebenprodukte. Durch eine einfache Destillation bei vermindertem Druck (50 mbar) wurden beide Reaktionsprodukte in guter Reinheit (jeweils über 98 %) gewonnen.

Der Umsatz an Diglykol lag bei 99 %, die Gesamtausbeute bei ca. 90 %.

Beispiel 4 (Vergleich)

Man führte die Umsetzung analog Beispiel 3, jedoch bei atmosphärischem Druck durch. Der Zulauf an Diglykol betrug 0,08 l pro l Katalysator und Stunde. Es ergab sich ein Umsatz an Diglykol von 85 % bei einer Gesamtausbeute von 70 %.

Erhöhte man den Zulauf an Glykol in Richtung auf den Wert des Beispiels 3, so sanken sowohl Umsatz wie auch Gesamtausbeute weiter ab.

Beispiel 5

N,N,N',N'-Tetramethyldiaminohexan
Es wurde die Anordnung des Beispiels 1 verwendet und wie folgt betrieben :

| | |
|---|---|
| Gesamtdruck : | 8 bar |
| Reaktionstemperatur | 210 °C |
| Kreisgasmenge | 500 Druckliter/l Katalysator und Stunde |
| Dimethylamingehalt im Kreisgas | |
| vor dem Reaktor : | 5 bis 7 Vol.% |
| nach dem Reaktor : | 2 bis 3 Vol.% |
| Zulauf zum Verdampfer : | Hexandiol-Lösung in Methanol mit 70 Gew.% Diol 0,4 l Lösung/l Katalysator und Stunde |

Fraktionierende Destillation und gaschromatographische Analysen ergaben für die Zusammensetzung des Kondensats :

| | |
|---|---|
| Reaktionswasser | 11 Gew.% |
| Methanol | 18 Gew.% |
| gelöstes Dimethylamin | 8 Gew.% |
| N-Methylhexamethylenimin | 4 Gew.% |

| | |
|---|---|
| Tetramethyldiaminohexan | 53 Gew.% |
| Dimethylaminohexanol | 1 Gew.% |
| Hexandiol | 0,5 Gew.% |
| Sonstige | 4 Gew.% |
| Destillationsrückstand | 0,5 Gew.% |

Durch eine einfache Destillation bei vermindertem Druck wurde das Zielprodukt in guter Reinheit (über 98 %) gewonnen.

Beispiel 6 (Vergleich)

Führt man die Reaktion bei atmosphärischem Druck durch, so erhält man ein ähnliches Ergebnis wie im Beispiel 5 bei folgenden Bedingungen :

| | |
|---|---|
| Reaktionstemperatur | 180 °C |
| Dimethylamingehalt im Kreisgas : | |
| vor dem Reaktor : | 10 bis 15 vol.% |
| nach dem Reaktor : | 5 bis 10 Vol.% |
| Zulauf zum Verdampfer : | 0,1 l Lösung/l Katalysator und Stunde |

Das Kondensat ist ähnlich zusammengesetzt wie im Beispiel 5, jedoch mit dem Unterschied, daß es bis zu 5 Gew.% nicht identifizierte hochsiedende Anteile (=Destillationsrückstand) enthält.

Erhöht man die Zulaufmenge über die angegebene Menge von 0,1 l/l.h hinaus, so steigt im Kondensat der Anteil von Dimethylaminohexanol und Hexandiol. Erhöht man außerdem die Temperatur, so steigt im Kondensat der Anteil von N-Methylhexamethylenimin und der hochsiedende Anteil, während gleichzeitig die Anteile von Dimethylaminohexanol und Hexandiol zurückgehen.

Diese « Tendenz-Analyse » soll zeigen, daß die Betriebsbedingungen zum Beispiel 6 in der Nähe eines relativen Optimums liegen, so daß Änderungen das Ergebnis verschlechtern, solange der Synthesedruck konstant bei Atmosphärendruck belassen wird.

Das Beispiel 6 zeigt außerdem einen Vorteil des erhöhten Druckes : bei gleichem Syntheseergebnis wie bei atmosphärischem Druck kann der Durchsatz vervielfacht werden.

Beispiel 7

N,N,N',N'-Tetramethyldiaminohexan

Es wurde vorgegangen wie im Beispiel 5, jedoch mit dem Unterschied, daß als Zulauf zum Verdampfer eine methanolfreie Hexandiolschmelze verwendet wird in einer Menge von 0,2 l pro l Katalysator und Stunde.

Fraktionierende Destillation und gaschromatographische Analysen ergaben für die Zusammensetzung des Kondensates :

| | |
|---|---|
| Reaktionswasser | 15 Gew.% |
| gelöstes Dimethylamin | 5 Gew.% |
| N-Methylhexamethylenimin | 5 Gew.% |
| Tetramethyldiaminohexan | 68 Gew.% |
| Dimethylaminohexanol | 1 Gew.% |
| Hexandiol | — |
| Sonstige | 6 Gew.% |
| Destillationsrückstand | — |

Beispiel 8 (Vergleich)

Es wurde vorgegangen wie im Beispiel 6, jedoch mit dem Unterschied, daß als Zulauf zum Verdampfer eine methanolfreie Hexandiolschmelze verwendet wird in einer Menge von 0,1 l pro l Katalysator und Stunde.

Fraktionierende Destillation und gaschromatographische Analysen ergaben für die Zusammensetzung des Kondensates nach 48 Stunden Betriebszeit :

| | |
|---|---|
| Reaktionswasser | 13 Gew.% |
| gelöstes Dimethylamin | 8 Gew.% |
| N-Methylhexamethylenimin | 4 Gew.% |
| Tetramethyldiaminohexan | 47 Gew.% |
| Dimethylaminohexanol | 14 Gew.% |
| Hexandiol | 1 Gew.% |
| Sonstige | 5 Gew.% |
| Destillationsrückstand | 8 Gew.% |

Mit fortschreitender Versuchsdauer veränderte sich das Kondensat ständig, bis der Anteil an Dimethylaminohexanol größer wird als der Anteil des erwünschten Tetramethyldiaminohexans.

Dieses Beispiel 8 macht einen weiteren Vorteil des erhöhten Druckes deutlich: Der bei atmosphärischem Synthesedruck zweckmäßige Zusatz von Methanol ist bei erhöhtem Druck nicht erforderlich.

## Patentansprüche

1. Verfahren zur Herstellung von Aminen der allgemeinen Formel I

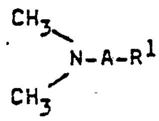

(I)

in der $R^1$ eine Hydroxylgruppe oder Dimethylaminogruppe und A eine Alkylengruppe mit 2 bis 6 Kohlenstoffatomen bedeuten, wobei die Kohlenstoffatomkette der Alkylengruppe gegebenenfalls durch Sauerstoffatome oder $N—R^2$-Gruppen, in denen $R^2$ für ein Wasserstoffatom oder eine niedere Alkylgruppe steht, unterbrochen sein kann, durch Umsetzung von Alkoholen der allgemeinen Formel II

$$HO—A—R^1 \qquad (II)$$

in der $R^1$ und A die oben genannte Bedeutung besitzen, mit Dimethylamin in der Gasphase an einem kupferhaltigen Katalysator, dadurch gekennzeichnet, daß man die Umsetzung bei einer Temperatur von 160 bis 240 °C und bei einem Druck von 5 bis 30 bar durchführt und dabei ein Molverhältnis Dimethylamin : Moläquivalent OH-Gruppen von 0,5 : 1 bis 3 : 1 einhält.

2. Verfahren gemäß Anspruch 1, dadurch gekennzeichnet, daß man ein Molverhältnis Dimethylamin : Moläquivalent OH-Gruppen von 1 : 1 bis 1,5 : 1 einhält.

3. Verfahren gemäß Anspruch 1, dadurch gekennzeichnet, daß man die Umsetzung bei einem Druck von 8 bis 25 bar durchführt.

4. Verfahren gemäß Anspruch 1, dadurch gekennzeichnet, daß man einen kupferhaltigen Katalysator verwendet, wie er durch Temperung eines basischen Kupfer und Aluminium enthaltenden Carbonats der allgemeinen Zusammensetzung

$$Cu_mAl_6(CO_3)_{0,5m}O_3(OH)_{m+12} ,$$

wobei m einen beliebigen, auch nicht ganzzahligen Wert zwischen 2 und 6 bedeutet, bei einer Temperatur von 350 bis 700 °C erhältlich ist.

## Claims

1. A process for preparing an amine of the general formula I

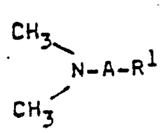

(I)

where $R^1$ is hydroxyl or dimethylamino and A is alkylene of from 2 to 6 carbon atoms which may be interrupted by oxygen or $—NR^2$, where $R^2$ is hydrogen or lower alkyl, by reacting an alcohol of the general formula II

$$HO—A—R^1 \qquad (II)$$

where $R^1$ and A are each as defined above, with dimethylamine in the gaseous phase over a copper-containing catalyst, which comprises performing the reaction at from 160 to 240 °C and from 5 to 30 bar while maintaining a molar ratio of dimethylamine : mole equivalent of OH groups of from 0.5 : 1 to 3 : 1.

2. A process as claimed in claim 1, wherein a molar ratio of dimethylamine : mole equivalent of OH groups of from 1 : 1 to 1.5 : 1 is maintained.

3. A process as claimed in claim 1, wherein the reaction is carried out at from 8 to 25 bar.

4. A process as claimed in claim 1, wherein the copper-containing catalyst used is obtainable by heating a basic copper/aluminum carbonate of the general composition

$$Cu_mAl_6(CO_3)_{0.5m}O_3(OH)_{m+12}$$

where m is any desired integer or non-integer from 2 to 6, at from 350 to 700 °C.

**Revendications**

1. Procédé de préparation d'amines de formule générale I

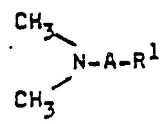

(I)

dans laquelle $R^1$ représente un groupement hydroxy ou diméthylamino et A un groupement alkylène ayant de 2 à 6 atomes de carbone, la chaîne d'atomes de carbone du groupement alkylène pouvant être éventuellement interrompue par des atomes d'oxygène ou des groupements N—$R^2$ où $R^2$ est mis pour un atome d'hydrogène ou un groupement alkyle inférieur, par réaction d'alcools de formule générale II

$$HO—A—R^1 \qquad (II)$$

dans laquelle $R^1$ et A ont les significations données ci-dessus, avec la diméthylamine en phase gazeuse sur un catalyseur contenant du cuivre, caractérisé en ce qu'on mène la réaction à une température de 160 à 240 °C et à une pression de 5 à 30 bar et on maintient en outre un rapport molaire diméthylamine : équivalent molaire de groupements OH de 0,5 : 1 à 3 : 1.

2. Procédé selon la revendication 1, caractérisé en ce qu'on maintient un rapport molaire diméthylamine : équivalent molaire de groupements de 1 : 1 à 1,5 : 1.

3. Procédé selon la revendication 1, caractérisé en ce qu'on mène la réaction à une pression de 8 à 25 bar.

4. Procédé selon la revendication 1, caractérisé en ce qu'on utilise un catalyseur contenant du cuivre qui a été préparé à une température de 350 à 700 °C par recuit d'un carbonate basique contenant du cuivre et de l'aluminium de constitution générale

$$Cu_mAl_6(CO_3)_{0,5m}O_3(OH)_{m+12} ,$$

où m représente une valeur quelconque mais non entière entre 2 et 6.